# EUROPEAN PATENT SPECIFICATION

(11) **EP 1 725 244 B1**
(45) Date of publication and mention of the grant of the patent: **29.12.2010**
(21) Application number: 05713495.9
(22) Date of filing: 15.02.2005
(51) Int. Cl.: A61K 31/727, A61K 38/36, A61K 38/55, A61P 11/00

(54) **METHOD OF PREVENTING FIBRIN CLOTS IN PULMONARY TISSUE THROUGH THE USE OF AEROSOLIZED ANTICOAGULANTS**
VERFAHREN ZUR VERHINDERUNG VON FIBRINGERINNSELN IM LUNGENGEWEBE DURCH VERWENDUNG VON AEROSOLISIERTEN GERINNUNGSHEMMERN
METHODE PERMETTANT DE PREVENIR LA FORMATION DE CAILLOTS DE FIBRINE DANS LE TISSU PULMONAIRE, CONSISTANT A UTILISER DES ANTICOAGULANTS EN AEROSOL

(30) Priority: 20.02.2004 US 546236 P; 14.02.2005 US 57522
(43) Date of publication of application: 29.11.2006
(60) Divisional application: 10177819.9
(73) Proprietor: Board of Regents, The University of Texas System, Austin, TX 78701 (US)
(72) Inventor: ENKHBAATAR, Perenlei, Galveston, TX 77551 (US); MURAKAMI, Kazunori, Apt. 3512, Galveston, TX 77551 (US); TRABER, Daniel, L., Galveston, TX 77551 (US)
(74) Representative: Williams, Gareth Owen
(86) International application number: PCT/US2005/004604
(87) International publication number: WO 2005/081816

(56) References cited:
- WO-A2-03/084476
- US-A- 5 843 705
- US-A- 6 124 257
- US-A- 6 127 347
- US-A1- 2002 128 225
- US-A1- 2003 007 966
- US-A1- 2003 124 705
- MURAKAMI KAZUNORI ET AL: "Heparin nebulization attenuates acute lung injury in sepsis following smoke inhalation in sheep." SHOCK (AUGUSTA, GA.) SEP 2002, vol. 18, no. 3, September 2002 (2002-09), pages 236-241, XP002534131 ISSN: 1073-2322
- ABUBAKAR K ET AL: "Heparin improves gas exchange during experimental acute lung injury in newborn piglets." AMERICAN JOURNAL OF RESPIRATORY AND CRITICAL CARE MEDICINE NOV 1998, vol. 158, no. 5 Pt 1, November 1998 (1998-11), pages 1620-1625, XP002534132 ISSN: 1073-449X
- WARREN BRIAN L ET AL: "HIGH-DOSE ANTITHROMBIN III IN SEVERE SEPSIS: A RANDOMIZED CONTROLLED TRIAL" JAMA THE JOURNAL OF THE AMERICAN MEDICAL ASSOCIATION, AMERICAN MEDICAL ASSOCIATION, US, vol. 286, no. 15, 17 October 2001 (2001-10-17), pages 1869-1878, XP009074599 ISSN: 0098-7484
- CHAN A ET AL: "Covalent antithrombin-heparin complexes with high anticoagulant activity. Intravenous, subcutaneous, and intratracheal administration." THE JOURNAL OF BIOLOGICAL CHEMISTRY 29 AUG 1997, vol. 272, no. 35, 29 August 1997 (1997-08-29), pages 22111-22117, XP002534133 ISSN: 0021-9258
- UCHIBA M. ET AL.: 'Effects of various doses of antithrombin III on endotoxin-induced endothelial cell injury and coagulation abnormalities in rats' THROMBOSIS RESEARCH vol. 89, 1998, pages 233 - 241, XP002973224

## Description

### BACKGROUND

In inflammatory lung diseases, fibrin is observed in the airways. Alveolar fibrin formation is known as a hallmark of acute and/or chronic inflammatory lung diseases. Plasma transudates enter into the airways in inflammatory condition because of increase in pulmonary vascular permeability. Fibrinogen in the exuded plasma is activated by tissue factor expressed on surface of activated alveolar macrophages and epithelial cells, which thereafter results in clot formation in the airways. Intrabroncheal fibrin plays several roles in the pulmonary pathology. First, it blocks ventilation. According to the current invention we have shown that more than 40% of bronchi and bronchioles were obstructed by fibrin-containing cast after smoke inhalation and pneumonia in sheep. When some part of lung is obstructed, gas exchange falls because of ventilation / per mismatch. If the patients were mechanically ventilated, the ventilated part of lung would be overstretched and it would be a cause of barotraumas. Secondly, fibrin inhibits surfactant activity also limiting lung function. It is known that heparin nebulization is effective to prevent airway obstruction and subsequent acute lung injury. However, there is a report suggesting that the administration of heparin alone, in a nebulize form, was not effective in smoke inhalation-induced acute lung injury (ALI). We think that the reason for this discrepancy is because heparin's anti-coagulant potential is antithrombin dependent and the antithrombin activity was not properly taken into account in other work, and that in this injury model antithrombin levels typically drop well below normal physiologic levels.

Another type of lung injury ARDS (acute respiratory distress syndrome) is a severe form of lung injury that occurs in approximately one tenth of U.S. critical care patients, it is a major contributor to ICU morbidity and mortality. The pathophysiology of ARDS includes inflammatory, vascular leak, uncontrolled coagulation and fibro-proliferative components. Local expression of thrombin and factor Xa enzymatic activity in the early ARDS lung causes intra-alveolar fibrin deposition and hyaline membrane formation that then contribute to impaired pulmonary function. Through its growth factor activity thrombin may also promote the development of lung fibrosis, which is a problem in later stages of ARDS. According to the current invention, models of acute lung injury have been used to investigate the role that thrombin plays in ARDS evolution and in its progression to multiple organ failure and eventual death in many patients.

Antithrombin is a broad-spectrum serine protease inhibitor. ATIII is a serpin with potent anti-coagulant activity. Rates of ATIII-mediated thrombin and factor Xa inhibition increase over 1000-fold upon binding to pharmaceutical heparin or vascular heparan sulfate proteoglycans. ATIII inhibits thrombin by forming a thrombin-ATIII complex that is removed from the circulation by the reticuloendothelial system. ATIII has further inhibitory action on all known coagulation pathways. ATIII can also, together with heparin to inactivate TF-factor VII complexes. AT (antithrombin III) inhibits thrombin and fibrin formation and reduces the expression of adhesion molecules by endothelial cells. APC (activated protein C) inhibits F VIIIa and F Va, reduces endothelial cells and monocyte TF expression, inhibits PAI-1 and TAFI (thrombin-activatable fibrino-lysis inhibitor) by reducing thrombin generation. Recombinant human soluble thrombomodulin (rhs-TM) is able to convert protein C in its activated form (APC).

ATIII contains a heparin-binding domain at its active site. Heparin, other glycos-aminoglycans, and proteoglycans are expressed on endothelial surfaces and bind ATIII with high affinity. To inactivate thrombin, ATIII must bind to heparin. In the absence of heparin, AT binds to endothelial surfaces and functions as an anticoagulant, but it also stimulates prostacyclin release. Prostacyclin is a potent inhibitor of platelet aggregation, inhibits synthesis of pro-inflammatory cytokines,and attenuates neutrophil activation and subsequent release of elastase and oxygen free radicals.

Heparin accelerates antithrombins' inhibitory reaction several thousand fold. Thus, without proper levels of antithrombin in the airways, aerosolized heparin would not inhibit fibrin formation leading to little if any improvement in treatment of lung injury by smoke or bums. According to the current invention we demonstrate that antithrombin nebulization in conjunction with heparin delivery is an effective and reliable in treatment for ALI from a smoke and/or bum induced injury and claim methods of treating such lung injuries more effectively.

Several human and animal studies have examined the effects of modulating the coagulation cascade in acute and chronic lung disease. According to the current invention, the exogenous delivery of the highly specific direct thrombin inhibitor, antithrombin III, is protective in animal models of ALI. In addition, heparin, which inhibits coagulation proteases by potentiating the formation of antithrombin III-serine protease complexes, leads to improved gas exchange in an animal model of ALI. Heparin has also been shown to attenuate bleomycin-induced pulmonary fibrosis in mice. Therefore concurrent administration of the two molecules has a synergistic and positive effect on ALI patients.

In LPS-induced lung injury, intravenous ATIII (250 units/kg) has been shown to reduce vascular injury, leukocyte accumulation, and vascular permeability. Heparin, alone may not be able to fully or optimally prevent lung damage. ALI and ARDS are associated with increased pro-coagulant and reduced fibrinolytic activities in alveoli and interstitial spaces in the lung. Fibrin deposition, which is the hallmark of early phase ALI, is accompanied by increased levels of PAI-I and neutrophil accumulation in the lung. Fibrin deposition and neutrophil elastase-induced digestion to fibrin degradation products stimulate fibroblast aggregation and collagen secretion, leading to pulmonary fibrosis. Whereas animal studies have demonstrated a consistent reduction in lung injury with the administration of anti-coagulants, such as TFPI, TF-factor VIIai, ATIII, soluble TM, and APC, clinical studies have been less convincing. ATIII administration has shown some benefits on lung injury due to smoke inhalation and bums.

Airway obstruction by fibrin cast is a serious problem in the smoke inhalation-induced acute lung injury (ALI). Prevention of airway casts may be important for clinical outcome. We report herein that ATIII and heparin jointly administered is an effective to prevent airway obstruction in an ovine smoke inhalation model. Moreover, the current invention teaches that the use of both heparin and antithrombin (ATIII), in a nebulized form and used for the treatment of lung injury act synergistically to improve gas exchange during experimental respiratory distress syndrome. ATIII and Heparin may also be useful for treating sepsis-induced coagulopathy. As previously stated heparin potentiates the naturally occurring plasma protease inhibitor, antithrombin, to inhibit thrombin, factor Xa, and other coagulation enzymes. When ATIII is supplied along with heparin this combination acts synergistically to inhibit thrombin and fibrin deposition.

US 2003/007966 discloses the use of ATIII administered by inhalation for treatment of acute lung injury in a subject

### SUMMARY OF THE INVENTION

The invention is based, in part, on the discovery that aerosolized antithrombin III (ATIII) is effective in treating lung disorders, e.g., lung inflammation and injury. Through the instant invention it has been determined that the prevention of the fibrin clots by aerosolized anticoagulants improves pulmonary lung function with burn and smoke inhalation injuries.

Thus, administration of both heparin and ATIII by Inhalation provides more efficient treatment of lung disorders, e.g., lung inflammation and injury, than intravenous administration.

According to the current invention there is provided the use of antithrombin III and heparin in the manufacture of a medicament for the treatment of acute lung injury wherein the medicament is administered in a therapeutically effective amount by inhalation. Aerosolized versions of both antithrombin and heparin were found to be a more effective anticoagulant to prevent the airway obstruction than heparin or other agents alone. This was also found to be true when aerosolized antithrombin and heparin were tested against a synthetic thrombin inhibitor, in smoke inhalation induced ALI model in sheep.

Both antithrombin and heparin nebulization inhibited the airway obstruction and attenuated the gas exchange. In addition to that, antithrombin seems to have an anti-inflammatory effect, which results in the attenuation of lung inflammation and subsequent edema formation when challenged with smoke and/or burn injury.

Accordingly, in one aspect, described herein is a method of treating a subject having a lung disorder, e.g., lung inflammation and/or injury, which Includes administration of a therapeutically effective amount of both ATIII and heparin by inhalation. The lung disorder can be an acute or chronic lung disorder. In one embodiment, the lung disorder is an acute lung injury, e.g., septic acute lung injury or ARDS, or airway blockage. Lung injury and/or inflammation can be in response to, e.g., exposure to an external agent, e.g., a viral agent (e.g., Pseudomonas pneumonia), smoke or asbestos. In other embodiments, the lung disorder can be, e.g., lung or pleural neoplasia, interstitial lung disease and/or organizing pteuitis.

In one embodiment, the composition of heparin and ATIII is administered using a jet aerosol or ultrasonic nebulizer system, or by a dry powder inhalation system. Such systems for aerosol administration of combination compositions are known,

In one embodiment, the ATIII is human ATIII. The ATIII can be naturally derived, e.g., from plasma, or recombinantly produced. Plasma derived ATIII is commercially available. In a preferred embodiment, the antithrombin III is transgenically produced, e.g., the ATIII is obtained from milk from a transgenic diary animal, e.g., a cow, a goat, a rabbit, or a mouse. Methods of producing ATIII in the milk of a transgenic animal are described in U.S. Patent Number 5,843,705 .Likewise, heparin can be naturally derived, e.g., from plasma, or recombinantly produced. Plasma derived heparin is commercially available. In a preferred embodiment, the heparin is produced recombinantly.

In a preferred embodiment, the subject is administered an aerosol composition that-includes ATIII, heparin and a pharmaceutically acceptable carrier. Examples of pharmaceutically acceptable carriers include water and saline.

In one embodiment, the subject is periodically administered both ATIII and heparin by inhalation, e.g., the subject is administered ATIII and heparin at regular intervals depending upon the pharmokinetics of each molecule. For example, the subject can be administered aerosol ATIII and heparin at the onset of lung inflammation and/or injury and then at set intervals after the initial administration, e.g., ATIII and/or heparin can be administered by inhalation every hour, 2 hours, 3 hours, 4 hours, 6 hours, twice a day, or three, four, five, six times a day dependent upon the patients need and the pharmacologically profile of the individual agents. The period of administration can be over a period of about 24, 48, 72, 96, 120, 144 or 168 hours. In another embodiment, the subject is administered the ATIII and heparin combination by inhalation as needed, e.g., ATIII is administered upon indication of one or more continued or reoccurring symptom(s) of lung inflammation or injury.

An effective dose of ATIII, e.g., transgenically produced ATIII, can be between about 1-150 U/kg, 25-125 U/kg, 50-100 U/kg, or 60-75 U/kg of body weight. In another aspect, an effective dose can be greater than about 1 mg/kg, 5 mg/kg, 10 mg/kg, but less than about 150 mg/kg, 100 mg/kg, 70 mg/kg.

An effective dose of the companion composition heparin, can be between about 1-30 U/kg, 2-25 U/kg, 50-100 U/kg, or 60-75 U/kg of body weight. In another aspect, an effective dose can be greater than about 1 mg/kg, 5 mg/kg, 10 mg/kg, but less than about 150 mg/kg. 100 mg/kg, 70 mg/kg.

In a preferred embodiment, the dose of aerosol for the ATIII & heparin combination is less than 10%, 20%, 30%. 40%, 50%, 60% the dose of ATIII & heparin intravenously administered to treat the same disorder, e.g., to have the same effect on one or more symptom of lung inflammation or injury.

According to the current invention both antithrombin and heparin nebulization inhibited the airway obstruction and attenuated the gas exchange. In addition to that, antithrombin had an additional and enhanced anti-inflammatory effect, which results in the improved attenuation of lung inflammation and subsequent edema formation. Moreover, there is a synergistic effect when antithrombin and heparin are used together, to treat lung injury due to smoke inhalation and/or bums.

Also described herein is a kit for treating lung injuries. Preferably, the kit includes a therapeutically effective amount of an aerosol form of ATIII, and instructions for use. Preferably, the aerosol further includes a pharmaceutically acceptable carrier. Examples of pharmaceutically acceptable carriers include water and saline.

In one embodiment, an effective dose of ATIII, e.g., transgenically produced ATIII, can be between about 10-300 U/kg, 25-125 U/kg, 50-100 U/kg, or 60-75 U/kg of body weight. In another aspect, an effective dose can be greater than about 1 mg/kg, 5 mg/kg, 10 mg/kg, but less than about 150 mg/kg, 100 mg/kg, 70 mg/kg.

In a preferred embodiment, the kit is a kit for treating an acute or chronic lung disorder. Preferably, the lung disorder is an acute lung injury, e.g., septic acute lung injury or acute respiratory distress syndrome (ARDS). Lung injury and/or inflammation can be in response to, e.g., exposure to an external agent, e.g., a viral agent (e.g., *Pseudomonas pneumonia*), smoke or asbestos. In other embodiments, the lung disorder can be, e.g., lung or pleural neoplasia, interstitial lung disease and/or organizing pleuitis.

In one embodiment, the kit includes the ATIII & heparin composition in a jet aerosol or ultrasonic nebulizer system, or a dry powder inhalation system.

In one embodiment, the kit includes an aerosol forum of human ATIII and heparin. The ATIII can be naturally derived, e.g., from plasma, or recombinantly produced. In a preferred embodiment, the antithrombin III is transgenically produced, e.g., the ATIII is obtained from milk from a transgenic diary animal, e.g., a cow, a goat, a rabbit, or mouse. This while the heparin of the invention is typically derived from a recombinant *in vitro* source.

The details of one or more embodiments of the invention are set forth in the accompanying drawing and the description below. Other features, objects, and advantages of the invention will be apparent from the description and drawings, and from the claims.

### DESCRIPTION OF DRAWINGS

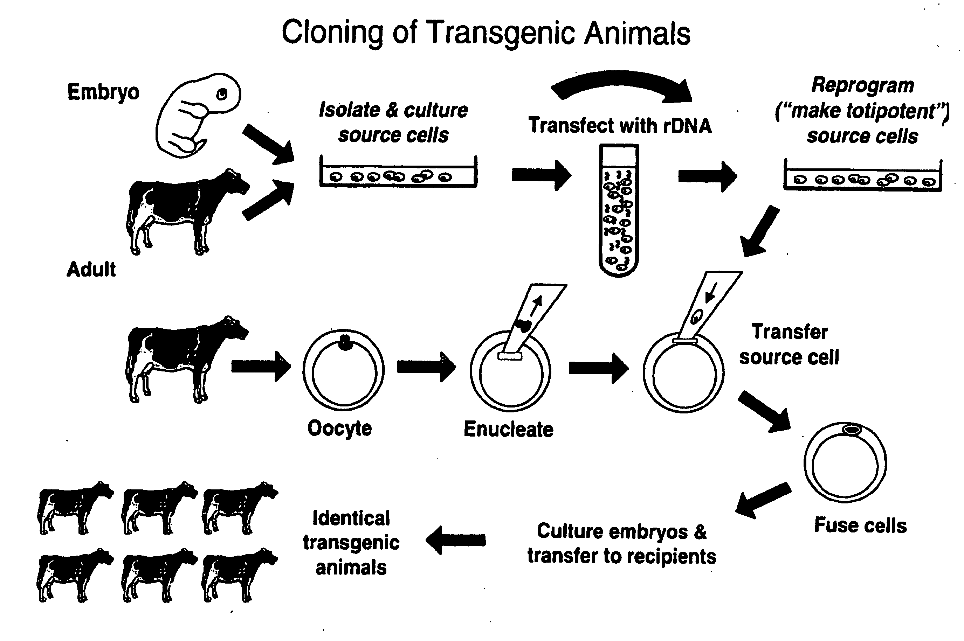

FIG. 1 Shows a Generalized Diagram of the Process of Creating Cloned Animals through Nuclear Transfer.

### DETAILED DESCRIPTION

According to the present invention, it was found that the use of an aerosol form of ATIII & Heparin reduced ALI at lower doses than intravenously administered ATIII alone or beparin alone. Accordingly, the invention features aerosol formulations including ATIII, as well as, methods of using such aerosol forms of ATIII to treat a subject having a lung disorder, e.g., lung injury or inflammation.

Recombinant human ATIII and heparin nebulized and delivered together, were an effective in preventing airway obstruction as well as improving the gas exchange after smoke inhalation injury followed by pneumonia. Further more, ATIII reduced the lung inflammation and subsequent edema formation. The amount of ATIII was less than that effective in intravenous administration. According to the data presented herein and according to a preferred embodiment of the instant invention there was no adverse effect observed including bleeding tendency. Therefore the use of aerosolized ATIII & heparin nebulization is a new and more effective treatment strategy for the treatment of smoke inhalation than either compound alone.

The term "treat" or "treatment" as used herein refers to alleviating or reducing one or more symptom(s) associated with a lung disorder. For example, symptoms of lung injury and/or inflammation include: 1) reduced pulmonary gas exchange; 2) reduced pulmonary shunt fraction; 3) extracellular fibrin deposition; 4) increased vascular permeability; 5) decreased lipoprotein surfactant deposition; 6) tissue remodeling:7) coagulation; and/or 8) increased alveolar tension. As used herein. an amount of an aerosolized form of ATIII effective to treat a lung disorder, or a "therapeutically effective amount" refers to an amount of ATIII aerosol which is effective, upon single or multiple dose administration to a subject, in curing, alleviating, relieving or improving a subject with a lung disorder as described herein beyond that expected in the absence of such treatment.

The ATIII & heparin combination can be administered as a dry powder formulation, or with a pharmaceutically acceptable carrier. Pharmaceutically acceptable carriers include, e.g., sterile water, saline and alcohols. The pharmaceutical ATIII & heparin aerosol composition can further include other therapeutic agents (e.g., other agents which alleviate or reduce lung inflammation or injury), or other pharmaceutical adjuvants, diluents, etc. The composition of the invention can be administered, e.g., as a complex with, or encapsulated in a liposome.

For administration by inhalation, the compounds used in the invention can be delivered in the form of an aerosol spray from pressured container or dispenser which contains a suitable propellant, e.g., a gas such as carbon dioxide, or a nebulizer. As used herein, the term "aerosols" refers to dispersions in air of solid or liquid particles, of fine enough particle size and consequent low settling velocities to have relative airborne stability (**See** Knight, V., VIRAL AND MYCOPLASMAL INFECTIONS OF THE RESPIRATORY TRACT. 1973, Lea and Febiger, Phila. Pa., pp. 2).

The nebulization of ATIII or heparin may be achieved by a gas pressure or by ultrasound. Generally speaking, a nebulizer is an apparatus permitting the administration of aerosols. The nebulizers may be of any type and their structures are known to a person skilled in the art, and these devices are commercially available. The aerosols of the invention can be made by nebulizing an ATIII & heparin containing solution using a variety of known nebulizing techniques. One nebulizing system is the "wo-phase" system which consists of a solution or a suspension of active ingredient in a liquid propellant. Both liquid and vapor phases are present in a pressurized container and when a valve on the container is opened, liquid propellant containing the solution or suspension is released. This can result in fine aerosol mist or aerosol wet spray.

There are a variety of nebulizers that are available to produce aerosols including small volume nebulizers. Compressor driven nebulizers incorporate jet technology and use compressed air or medical oxygen to generate the aerosol. Commercially available devices are available from Healthdyne Technologies Inc; Invacare Inc.; Mountain Medical Equipment Inc.; Pari Respiratory Inc.; Mada Medical Inc.; Puritan-Bennet; Schuco Inc.; Omron Healthcare Inc.; DeVilbiss Health Care Inc; and Hospitak Inc. Ultrasonic nebulizers, e.g., an ultrasonic type nebulizer with a quartz crystal vibrating at high frequency, can also be used to deliver the ATIII // heparin composition.

Toxicity and therapeutic efficacy of such ATIII aerosols can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

Other methods of determining the dosage of an ATIII & heparin joint composition will include measuring a subject's circulating ATIII levels prior to treatment with ATIII. Based on circulating ATIII levels, the dosage of ATIII can be adjusted to be 50%, 100%, 150%, 250%. 300% greater than initial circulating levels.

The amount of aerosol formulation administered will typically in the in range of about 10 U/kg to about 250 U/kg of body weight, preferably about 25 U/kg to about 175 U/kg of body weight.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

### MATERIALS AND METHODS

### Materials

Human recombinant antithrombin (ATIII) was a gift from GTC Biotherapeutics (Framingham, MA). Recombinant heparin was purchased from Daiichi Pharmaceutical Co. (Tokyo, Japan). All other reagents were of analytical grade.

### Animal preparation

The Animal Care and Use Committee of The University of Texas Medical Branch approved the experiments reported in this manuscript. All animals were handled within the guidelines established by the American Physiological Society and the National Institutes of Health. Twenty four female sheep were surgically prepared for study under halothane anesthesia as previously described. After measurement of baseline cardiovascular data, sheep received a combination injury of smoke inhalation and bacteria instillation as previously described. This animal model shows severe acute lung injury as well as hyperdynamic cardiovascular responses, which mimics human septic cases.

Briefly, *P. aeruginosa* (5 x 10⁹/kg) was instilled intrabronchially after the cotton smoke exposure (4 sets of 12 breaths, < 40 °C). After the smoke inhalation and bacterial instillation, animals were randomly assigned for the two treatment groups, ATIII & Heparin nebulization group; ATIII nebulization group, heparin-nebulization group, and saline nebulization control group. All animals were mechanically ventilated with 100% oxygen throughout the 24-h study (Tidal volume 12-15ml/kg, 30 breaths/min, PEEP 7.5cmH₂O). Tidal volume was adjusted to maintain PaCO₂ normal. The Ringer's lactate solution was injected in sufficient volume to prevent the hematocrit from rising. Initially, 2 ml/kg/h of Ringer's lactate was infused, and we adjusted the in rate up to 10 ml/kg/h based on the hematocrit levels. The body weight (kg) of animals did not significantly vary from one animal to another.

### Treatment

Recombinant human antithrombin (rhAT) and recombinant heparin were dissolved in distilled water. Then, the ATIII/heparin solution was mixed with saline and mixed solution containing ATIII & heparin and the animal was nebulized every 4 hrs. ATIII and Heparin was were separately mixed with saline and were also nebulized every 4 hrs. The control group received 15 ml saline nebulization as the same fashion. The solutions were nebulized by an ultrasonic nebulizer (Ultra-Neb99, DeVilbiss Co., Somerset, PA), which was connected to the tracheobronchial tree as shown previously. The size of the particles obtained by the nebulizer is reported less than 4 microns. The nebulization was started one hour after the insult and every 4 hrs thereafter.

### Measurement of plasma NO₂l NO₃ (NOx).

The concentration of NOx (total amount of nitric oxide metabolites) in plasma was measured with a chemiluminescent NO analyser (Antek, model 7020) as previously described.

### Blood culture and Coagulation parameters

Arterial blood was cultured to test the bacteremia (Septi-check, Becton Dickinson, Sparks, MD). Heparinized blood samples were carefully withdrawn from the femoral artery catheter at baseline and at 3, 6, 12, and 24 hrs.

Activated clotting time was monitored at baseline, 12h and 24h after the injury using Hemochron model 801 (International Technidyne Co., Edson, NJ).

Citrated plasma was collected at baseline, 6h, and 24h after the injury for the antithrombin assay. The activities of antithrombin was measured using a chromogenic substrates (S-2765, Diagnostica Stago, Parsippany, NJ). Data were expressed as % of normal standard plasma.

### Airway pressures

Peak and plateau airway pressures were monitored by a ventilator (Servo Ventilator 900C, Seimens-Elema, Sweden). Since peak airway pressure reflects the sum of airway resistance and compliance, and the plateau pressure reflects mainly the airway compliance, we subtracted the plateau pressure from the peak pressure (Δ airway pressures). Thus, Δ airway pressure is an index of airway resistance.

### Statistical analysis

The statistical software StatView 5.0 (SAS Institute, Cary, NC) was used to perform the analysis. Data are expressed as means ± SEM. Repeated measured analysis of variance (ANOVA) or ANOVA with Tuckey's post hoc test was used to compare data among the groups. Where appropriate, inter-group comparisons at individual time points were made using the Student Newman-Keuls test for unpaired data. In the histological study, a non-parametric Mann-Whitney U test was performed. A p-value < 0.05 was considered statistically significant.

### Methods for Joint Composition

Female sheep were surgically prepared for chronic study. After recovery, a tracheostomy was performed under ketamine/halothane anesthesia, and given a bum (40% of total body surface, 3^{rd} degree) and inhalation injury (cotton smoke). Sheep were given 4 ml/kg/24h of Ringer's lactate and mandatory ventilation. Sheep were divided into 5 groups: 1) non-injured, non-treated (sham, n=6); injured, nebulized with 2) saline (saline, n=6), 3) heparin (Hep, n=5), 4) antithrombin (AT, n=5), and hep+AT (n=5). Saline, heparin (10,000u), and antithrombin (290u) were nebulized every 4h beginning 2h after the injury. Experiment lasted 48h.

### Results and Conclusion

The cardiopulmonary variables were stable in sham animals. The saline group showed marked signs of acute lung injury evidenced by deteriorated gas exchange [PaO₂/FiO₂, pulmonary shunt (Qs/Qt)], increased lung lymph flow associated with increased airway pressures. Post treatment with heparin or antithrombin alone had no noticeable effects on these changes. However, combined heparin and antithrombin therapy reversed those alterations. The antithrombin concentration in bronchi-alveolar lavage in saline nebulized sheep was decreased. Taken together, the results indicated that administration of the joint composition prevented fibrin clot retention and relieved airway obstruction thereby improving airway clearance, improving pulmonary function in sheep with combined burn and smoke inhalation injury. Antithrombin and heparin exert stronger effect when they are aerosolized as a combination, See Table I below.

**Table I.**

| | Sham | Saline | Heparin | AT | Heparin+AT |
|---|---|---|---|---|---|
| PaO2/FiO2 | | | | | |
| 0h | 510±9 | 517±14 | 533±8 | 512±12 | 520±7 3 |
| 48h | 560±60 | 149±31† | 151±22† | 145±27† | 04±53*† |

| Qs/Qt | | | | | |
|---|---|---|---|---|---|
| 0h | 0.8±0.01 | 0.13±0.03 | 0.15±0.01 | 0.15±0.02 | 0.19±0.02 |
| 48h | 0.12±0.01 | 0.38±0.06† | 0.34±0.01† | 0.44±0.05† | 0.22±0.03* |

| | | | | | |
|---|---|---|---|---|---|
| *p<0.05 vs. saline; †<0.05 vs. sham; | | | | | |

### RESULTS

The peak levels of carboxy-hemoglobin (%) in the saline-treated group, ATIII-treated group, and heparin-treated group suggested that all the groups received almost the same amount of smoke inhalation. No animals died during the 24-h study period.

### Blood Culture

The arterial samples were carefully withdrawn for the bacterial culture at the several time points during the study as indicated in the Method section. *P*. *aeruginosa* was negative in all the baseline samples. During the 24h study, *P*. *aeruginosa* was detected in 33% (3/10) in saline-control group but 0% in both ATIII and heparin groups. Although the percentage was lower in ATIII and heparin-treated groups, there were no statistical differences from the saline-treated group.

### Pulmonary gas exchange

The PaO₂/FiO₂ (P/F) ratio dropped significantly after the insult in all the groups. However, the drop was significantly less in rhAT-nebulization group and heparin-nebulization group. The attenuation in gas exchange by ATIII and heparin was about the same extent. The pulmonary shunt fraction significantly increased after the insult, but the increase was significantly less in ATIII or heparin nebulization groups.

### Airway pressures

Peak and plateau airway pressures were monitored by the ventilator during the study. Delta airway pressure (=[Peak pressure]-[Plateau pressure]) was calculated to analyze the airway resistance. Delta airway pressure gradually increased in saline-treated control. ATIII-treated group and heparin-treated group showed no increase in delta airway pressures, although there were not statistical differences between the groups.

### Lung histopathology

Twenty-four hours after the smoke and bacterial challenge, there was a marked inflammatory reaction in the affected sheep's lungs that was characterized by cellular infiltrates in the interstitium and the air spaces. The infiltrates were predominantly composed of neutrophils. Interstitial edema, vascular congestion and hemorrhage were also observed. The histology scores, based on the severity and extent of congestion, edema, inflammation, and hemorrhage, were significantly higher after exposure to smoke and bacteria. Our historical sham-injured sham-treated animal (n=7) showed 1.0 or less in all the individual histopathology scores.

Heparin stone showed almost the same scores as saline control. Interestingly, hemorrhage score was not higher but less in both ATIII-treated and heparin-treated group. The total histology score was calculated. Since each congestion, edema, inflammation, and hemorrhage was scored from 0 (normal) to 4 (intense), the maximum of total histology score was 16. It was significantly lower in ATIII-treated group. In contrast, heparin nebulization did not attenuate the total histology score. It was 3.3±0.7 in historical sham-injury group (n=7).

Bronchi and bronchioles were obstructed by infiltrates of neutrophils, shed bronchial epithelial cells, mucus and fibrin. Airway obstruction of more than 30% of the cross-sectional area was found at both the bronchial and bronchiolar levels in saline treated group. ATIII nebulization inhibited the airway obstruction significantly in bronchi levels. Heparin inhibited the obstruction significantly in both bronchi and bronchiole levels.

### Lung wet/dry weight ratio

Lung wet/dry weight ratios increased significantly 24 h after smoke inhalation followed by bronchial instillation of bacteria compared to the historical sham injury's data (wet/dry ratio=3.70±0.17, n=7). This increase was significantly attenuated by ATIII nebulization but not by heparin alone.

### Changes in nitrate/nitrite (NOx) levels

Plasma NOx levels increased 2-3 folds of baseline levels after the insult. ATIII group showed the similar increase in initial 3h but significantly less in later phase until 24 h (Fig. 6). Heparin seemed to be lower in NOx levels during 6-15 hrs but it reached to the same extent as the saline-treated group later on. There was not a statistical difference between heparin-treated group vs. saline-treated group.

### Blood count and coagulation parameters

The baseline leukocyte and platelet numbers were not different between . the groups. The numbers of leukocytes fell significantly 24 h after the injury.

### DISCUSSION

Fibrin formation in the airway is known as a hallmark of acute/chronic lung injury. In septic condition, vascular permeability increases and fibrinogencontaining plasma comes into the airways, where tissue factors are expressed by the stimulation of cytokines. Therefore, fibrinogen easily makes a clot in the airways. Fibrin formation in the airway is a cause of lung injury by two means. First, it blocks the part of airways and inhibits the gas exchange. Second, fibrin inhibits the surfactant acti vity and it would be a cause of atelectasis. When the patients or animals are mechanically ventilated, the airway obstruction also causes ventilator induced lung injury. Also, overstretching the ventilated part of lung induces chemokines such as interleukin 8.

We have shown that heparin nebulization is effective in preventing airway obstruction and improving gas exchange. We determined that heparin nebulization prevents fibrin clot formation and attenuates acute lung injury. However, heparin's anticoagulant property is antithrombin dependent. According to the current invention, smoke inhalation, lung injury and pneumonia were initiated in laboratory animals designed to mimic what occurs in human occurrences of the same injuries or disease states. According to the current model, plasma antithrombin activity was measured and compared.

The baseline values of plasma antithrombin activity decreased gradually after the injury, and reached to approximately 50 % at 24 hrs. Similar phenomenon is reported in bum or sepsis patients. In such conditions, heparin's effect is not seen. The infusion of ATIII was effective in treating lung injury. Based on this intravenous study, it was determines that a lower dosage range of ATIII directly by a nebulizer into the airways would be effective as well. This is the reason we designed the present study. Also, we calculated the amount (moles) of aerosolized ATIII that we administered and tested the effect of heparin of approximately the same anti-thrombotic potential.

Antithrombin is one of the major physiologic anticoagulants. Besides inhibiting various coagulation factors such as thrombin, FXa, FXIa, FXIIa, and FIXa, antithrombin is known to have some anti-inflammatory aspects. Various animal and clinical studies showed that intravenous antithrombin was beneficial in sepsis, endotoxin shock, renal ischemia-reper injury, and burns. Two mechanisms of action are now considered. First, antithrombin promotes prostacyclin release from endothelial cells. Since prostacyclin inhibits cytokine production, adhesion molecule expression, and platelet aggregation, antithrombin inhibits the inflammatory reactions indirectly via prostacyclin. Second, antithrombin receptor called syndecan-4 was found recently. Once antithrombin binds to syndecan-4, it inhibits nuclear factor kappa B translocation and inhibits the inflammatory signal transduction in leukocytes and endothelial cells. Thus, antithrombin directly alters inflammatory processes via inhibition of NF-kappa B activation.

Heparin was designed to inhibit thrombin activity and the specificity is very high. Compare to antithrombin, heparin is a very small molecule (molecular weight is 527 Da) and directly inhibits thrombin. There are few reports showing the anti-inflammatory effect of heparin. We thought these differences between antithrombin and heparin might show different effects on inhalation injury. In the present study, both ATIII and heparin inhibited the airway obstruction following smoke inhalation and pneumonia almost to the same extent, see Table I. As a consequence, pulmonary gas exchange was attenuated by both ATIII and heparin. When used together they displayed a synergistic effect/ Regarding the airway obstruction, heparin was more effective in preventing cast formation than rhAT. Since heparin is smaller than ATIII and it is a non-protein material, it could be delivered better. However, according to the improvement afforded by the current invention the histopathological analysis showed that ATIII improved the histological changes but heparin did not. Consistent with this result, lung wet/dry weight ratio was improved by ATII nebulization but not by heparin. From these observations, we can speculate that anticoagulant potential of both ATIII and heparin inhibited the airway fibrin formation and attenuated the gas exchange. In addition to that, ATIII has an anti-inflammatory property and that improved the lung inflammation and subsequent lung edema formation. The drop in white cell count was less severe in ATIII-treated group compare to heparin-treated group, suggesting also that ATIII inhibited the inflammatory response.

Both ATIII and heparin also reduced the incidence of septisemia. When the airways are obstructed, bacterial clearance would be interrupted and the closed non-ventilated area would be the bacterial bed. Therefore, we think the inhibition of airway obstruction is beneficial not only for the gas exchange but also for the prevention of the systemic bacterial translocation and sepsis.

The increase in plasma NOx, an index of nitric oxide (NO) formation, was significantly suppressed by aerosolized rhAT. NO is synthesized from 3 kinds of isoforms of nitric oxide synthase (NOS). Endothelial NOS (eNOS) and neuronal NOS (nNOS) are known as constitutive NOS (cNOS) and are expressed all the time. The third isoform is called inducible NOS (iNOS). I NOS is induced by inflammatory cytokines such as tissue necrosis factor-alpha. In the present study, ATIII inhibited the NO formation especially the latter 12 h of the experiment but did not inhibit it in the initial 12 hrs, suggesting that ATIII inhibits the iNOS expression. We have shown that iNOS is expressed not only from the alveolar macrophages but also alveolar epithelial cells. Since antithrombin is reported to inhibit NFkB activation, we have determined that nebulized ATIII inhibits iNOS expression from airway epithelial cells.

**Table II**

| | Changes in blood counts | |
|---|---|---|
| Time | Baseline | 24hrs |
| WBC(x10³/µl). | | |
| Saline | 6.19±0.71 | 1.85±0.46* |
| rhAT | 4.71±0.54 | 2.96±1.12 |
| Heparin | 6.15±0.93 | 2.08±0.81* |

| Platelets (x10³/µL) | | |
|---|---|---|
| Saline | 447±93 | 276±65* |
| rhAT | 456±52 | 246±41* |
| Heparin | 417±t16 | 297±78 |

| | | |
|---|---|---|
| Mean ± SE. * p<0.05 vs. baseline | | |

**Table II Changes in activated clotting time**

| Time | Baseline | 24hrs |
|---|---|---|
| Saline | 151±8 | 168±7 |
| rhAT | 149±5 | 164±14 |
| Heparin | 147±2 | 157±5 |

| | | |
|---|---|---|
| Mean ± SE (sec) * p<0.05 versus saline | | |

Even though the total dose of ATIII was half of previously determined in intravenous studies (see Murakami (2001) Am.J. RESP. CRIT. CARE MED. 163:A553), the outcomes were more effective than intravenous administration. No adverse effects were observed. Thus, aerosolized ATIII was beneficial in septic acute lung injury following smoke inhalation and pneumonia in sheep.

A number of embodiments of the invention have been described. Other embodiments are within the scope of the following claims.

### Literature Cited

1. Abubakar K, Schmidt B, Monkman S, et al.. Heparin Improves Gas Exchange During Ex-Perimental Acute Lung Injury In Newborn Piglets. AM J RESPIR CRIT CARE MED 1998; 158:1620-1625.
2. Arocas V, Turk B, Bock SC, Olson ST, Bjork I (2000), The Region of Antithrombin Interacting with Full-Length Heparin Chains Outside the High-Affinity Pentasaccharide Sequence Extends to Lys 136 But Not to Lys 139. BIOCHEMISTRY 39:8512-8.
3. Desai U, Swanson R, Bock SC, Bjork I, Olson ST (2000), Role Of Arginine 129 In Heparin Binding And Acrivation Of Antithrombin. J. BIOL. CHEM. 275:18976-84.
4. Edmunds, T., S. M. Van Patten, J. Pollock, E. Hanson, R. Bernasconi, E. Higgins, P. Manavalan, C. Ziomek, H. Meade, J. M. McPherson, and E. S. Cole et al., 1998, Transgenically Produced Human Antithrombin: Structural And Functional Comparison To Humun Plasma-Derived Antithrombin. BLOOD 91(12):4561-71.
5. Gross, T. J., R. H. Simon, and R. G. Sitrin. 1992. Tissue Factor Procoagulant Expression By Rat Alveolar Epithelial Cells. AM J RESPIR CELL MOL. BIOL 6(4):397-403.
6. Herndon, D. N., D. L. Traber, G. D. Niehaus, H. A. Linares, and L. D. Traber 1984. The Pathophysiology Of Smoke Inhalation Injury In A Sheep Model. J TRAUMA 24(12): 1044-51.
7. Horie S, Ichii H, Kazama M. et al., Heparin-Likeglycosaminoglycan Is A Receptor For Anti-Thrombin III-Dependent But Not Thrombin-Dependent Prostacyclin Production In Humanendothelial Cells. THROMB RES 1990; 59:899 -904
8. Idell, S., A. P. Mazar, P. Bitterman, S. Mohla, and A. L. Harabin. 2001. Fibrin Turnover In Lung Inflammation And Neoplasia. AM J RESPIR CRIT CARE MED 163(2):578-84.
9. Isobe, H., K. Okajima, M. Uchiba, N. Harada, and H. Okabe. 2002. Antithrombin Prevents Endotoxin-Induced Hypotension By Inhibiting The Induction Of Nitric Oxide Synthase In Rats. BLOOD 99(5):1638-45.
10. Jesty J. Lorenz A, Rodriguez J, et al., Initiation Of Tissue Factor Pathway Of Coagulation In The Presence Of Heparin: Control By Anti-Thrombin III And Tissue Factor Pathway Inhibitor. BLOOD 1996; 15:2301-2307
11. Kaneider, N. C., P. Egger, S. Dunzendorfer, and C.I. Wiedermann. 2001. Syndecan-4 As Antithrombin Receptor Of Human Neutrophils. BIOCHEM BIOPHYS RES COMMUN 287(1):42-6.
12. Kancider, N. C., C. M. Reinisch, S. Dunzendorfer, J. Romisch, C. J. Wiedermann, and C. J. Wiederman. 2002. Syndecan-4 Mediates Antithrombin-Induced Chemotaxis Of Human Peripheral Blood Lymphocytes And Mlonocytes. J CELL SCI 115 (pt1):227-36.
13. Kimura, R., L. D. Traber, D. N. Herndon, G. D. Neuhaus, and D. L. Traber. 1988. Treatment Of Smoke-Induced Pulmonary Injury With Nebulized Dimethylsulfoxide. CIRC SHOCK 25(4):333-41.
14. Kimura R, Traber DL, Herndon DN, et al: Increasing Duration Of Smoke Exposure Induces More Severe Lung Injury In Sheep. J APPL PHYSIOL 1988; 64:1107-1113
15. Kowal-Vern, A., V. McGill, J. M. Walenga, and R. L. Gamelli. 2000. Antithrombin(H) Concentrate Infusions Are Safe And Effective In Patients With Thermal Injuries. J BURN CARE REHABIL 21(2):115-27.
16. Kowal-Vern, A., R. L. Gamelli, J. M. Walenga, D. Hoppensteadt, M. Sharp-Pucci, and H. R. Schumacher. 1992. The Effect Of Burn Wound Size On Hemostasis: A Correlation Of The Hemostatic Changes To The Clinical State. J TRAUMA 33(1):50-6; discussion 56-7.
17. McKeage, K., and G. L. Plosker. 2001. HEPARIN. DRUGS 61(4):515-22; discussion 523-4.
18. Minnema, M. C., A. C. Chang, P. M. Jansen, Y. T. Lubbers, B. M. Pratt, B. COS Whittaker, F. B. Taylor, C. E. Hack, and B. Friedman. 2000. Recombinant Human Antithrombin III Improves Survival And Attenuates Inflammatory Responses In Baboons Lethally Challenged With Escherichia Coli. BROOD 95(4):1117-23.
19. Mizutani, A., K. Okajima, M. Uchiba, H. Isobe, N. Harada, S. Mizutani, and T. Noguchi. 2003. Antithrombin Reduces Ischemia/Reperfusion-Induced Renal Injury In Rats By Inhibiting Leukocyte Activation Through Promotion Of Prostacyclin Production. BLOOD 101 (8):3029-36.
20. Murakami, K., L. J. Bjertnaes, F. C. Schmalstieg, R. McGuire, R. A. Cox, H. K. Hawkins, D. N. Herndon, L. D. Traber, and D. L. Traber. 2002. A Novel Animal Model Of Sepsis After Acute Lung Injury In Sheep. CRIT CARE MED 30(9):2083-90.
21. Murakami, K., R. McGuire, R. A. Cox, J. M. Jodoin, L. J. Bjertnaes, J: Katahira, L. D. Traber, F. C. Schmalstieg, H. K. Hawkins, D. N. Herndon, and D. L. Traber. 2002. Heparin Nebulization Attenuates Acute Lung Injury In Sepsis Following Smoke Inhalation In Sheep. SHOCK 18(3):236-41.
22. Murakami, K., R. McGuire, R. A. Cox, J. M. Jodoin, F. C. Schmalstieg, L. D. Traber, H. K. Hawkins, D. N. Herndon, and D. L. Traber. 2003. Recombinant antithrombin attenuates pulmonary inflammation following smoke inhalation and pneumonia in sheep. CRIT CARE MED 31 (2):577-83.
23. Oelschlager, C., J. Romisch, A. Staubitz, H. Stauss, B. Leithauser, H. Tillmanns, and H. Holschermann. 2002. Antithrombin III Inhibits Nuclear Factor kappaB Activation In Human Monocytes And Vascular Endothelial Cells. BLOOD 99(11):4015-20.
24. Olson ST, Bjork I, Bock SC (2002) Identification of Critical Molecular Interactions which Mediate Heparin Activation of Antithrombin: Implications for the Design of Improved Heparin Anticoagulants. TRENDS IN CARDIOVASCULAR MEDICINE.
25. Opal, S. M. 2000. Therapeutic Rationale For Antithrombin III In Sepsis. Crit Care Med 28(9 Suppl):S34-7.
26. Salvatierra A, Guerrero R, Rodriguez M, et al., Antithrombin III Prevents Early Pulmonary Dysfunction After Lung Transplantation In The Dog, CIRCULATION 2001; 104:2975-2980
27. Schmalstieg, F. C., J. Chow, C. Savage, H. E. Rudloff, K. H. Palkowetz, and J. B. Zwischenberger. 2001. Interleukin-8, Aquaporin-I, And Inducible Nitric Oxide Synthase In Smoke And Burn Injured Sheep Treated With Percutaneous Carbon Dioxide Removal. ASAIO J 47(4):365-71
28. Seeger, W., C. Grube, and A. Gunther. 1993. Proteolytic Cleavage Of Fibrinogen: Amplification Of Its Surfactant Inhibitory Capacity. AM J RESPITE CELL MOL BIOL 9(3):239-47.
29. Sitrin, R. G, P. G. Brubaker, J. E. Shellito, and H. B. Kaltreider 1986. The Distribution Of Procoagulant And Plasminogen Activator Activities Among Density Fractions Of Normal Rabbit Alveolar Macrophages. AM REV RESPIR DIS 133(3):468-72.
30. Soejima, K., L. D. Traber, F. C. Schmalstieg, H. Hawkins, J. M. Jodoin, C. Szabo, E. Szabo, L. Varig, A. Salzman, and D. L. Traber. 2001. Role Of Nitric Oxide In Vascular Permeability After Combined Burns And Smoke Inhalation Injury. AM J RESPIR CRIT CARE MED 163(3 Pt 1):745-52.
31. Tasaki, O., D. W. Mozingo, M. A. Dubick, C. W. Goodwin, L. D. Yantis, and B. A. Pruitt, Jr. 2002. Effects Of Heparin And Lisofylline On Pulmonary Function After Smoke Inhalation Injury In An Ovine Model. CRIT CARE MED 30(3):637-43.
32. Uchiba, M., K. Okajima, K. Murakami, H. Okabe, and K. Takatsuki. 1995. Effects Of Antithrombin III (AT III) And Trp49-Modified AT III On Plasma Level Of 6-Keto-PGFI Alpha In Rats. Thromb Res 80(3):201-8.
33. Uchida M, Okajima K, Murakami K: Effects Of Various Doses Of Antithrombin III And Endotoxin-Induced Endothelial Cell Injury And Coagulation Abnormalities In Rats. THROMB RES 1998; 89:233-241.
34. von Bethmann, A. N., F. Brasch, R. Nusing, K. Vogt, H. D. Volk, K. M. Muller, A. Wendel, and S. Uhlig. 1998. Hyperventilation Induces Release Of Cytokines From Perfused Mouse Lung. AM J RESPIR CRIT CARE MED 15 (1):263-72.
35. Vervloet, M. G., L. G Thijs, and C. E. Hack. 1998. Derangements Of Coagulation And Fibrinolysis In Critically III Patients With Sepsis And Septic Shock. SEMIN THROMB HEMOST 24(1):33-44.
36. Wiedermann Ch, J., and J. Romisch. 2002. The Anti-Inflammatory Actions Of Antithrombin--A Review. ACTA MED AUSTRIACA 29(3):89-92.
37. Yamanuchi T, Umeda F, Inoguchi I, et al., Antithrombin III Stimulates Prostacyclin Production By Cultured Aortic Endothelial Cells. BIOCHEM BIOPHYS RES COMMUN 1989;163:1404 -1411 S335 Crit Care Med 2003 Vol. 31, No. 4 (Suppl.).

## Claims

1. Use of antithrombin III and heparin in the manufacture of a medicament for the treatment of acute lung injury wherein the medicament is administered in a therapeutically effective amount by Inhalation.

2. The use of claim 1, wherein the lung injury is septic acute lung injury.

3. The use of claim 1. wherein the lung injury is acute respiratory distress syndrome (ARDS).

4. The use of claim 1, wherein the lung injury is in response to exposure to a viral agent.

5. The use of claim 1, wherein the lung injury is in response to a biological agent, and wherein the biological agent is Pseudomonas pneumonia.

6. The use of claim 1, wherein the lung injury is in response to one or more of smoke and asbestos.

7. The use of claim 1, wherein the antithrombin III and heparin combination is administered using an ultrasonic nebulizer.

8. The use of claim 1, wherein the antithrombin III is plasma derived antithrombin III.

9. The use of claim 1, wherein the antithrombin III is recombinantly produced antithrombin III.

10. The use of claim 9, wherein the recombinant antithrombin III is transgenically produced antithrombin III.

11. The use of claim 1, wherein the subject is administered more than one dose of the antithrombin III.

12. The use of claim 1, wherein the antithrombin III is administered at a dose of about 10-300 U/kg of body weight.

13. The use of claim 1, wherein the antithrombin III is administered at a dose of about 25-125 U/kg of body weight.

14. The use of claim 1, wherein the lung injury is treated both with ATIII and heparin.

15. The use of claim 1, wherein the lung injury treated was caused by smoke inhalation.

16. The use of claim 1, wherein the lung injury treated was caused by bum damage to the lungs.

17. The use of a protein being encoded by a transgene DNA construct comprising ATIII in the manufacture of a medicament for the treatment of acute lung injury.

18. The usa of a protein encodes by a transgeoe DNA construct comprising ATIII in the manufacture of a medicament for the treatment of acute lung injury wherein a substantially pharmaceutically pure composition of said protein is administered together with an effective amount of heparin.

19. The use of claim 1, wherein the heparin is plasma derived heparin.

20. The use of claim 1, wherein the heparin is recombinantly produced heparin.

21. The use of claim 20, wherein the recombinant heparin is transgenically produced heparin.

22. The use of claim 1, wherein the subject is administered more than one dose of the heparin.

23. The use of claim 1, wherein the heparin is administered at a dose of about 10-300 U/kg of body weight.

24. The use of claim 1, wherein the heparin is administered at a dose about 25-125 U/kg of body weight.

## Patentansprüche

1. Verwendung von Antithrombin III und Heparin bei der Herstellung eines Medikaments zur Behandlung von akuter Lungenverletzung, wobei das Medikament in einer therapeutisch wirksamen Menge durch Inhalation verabreicht wird.

2. Verwendung nach Anspruch 1, wobei die Lungenverletzung eine septische akute Lungenvetletzung ist.

3. Verwendung nach Anspruch 1, wobei die Lungenverletzung das akute Atemnotsyndrom (ARDS) ist.

4. Verwendung nach Anspruch 1, wobei die Lungenverletzung als Reaktion auf die Einwirkung eines viralen Agens auftritt.

5. Verwendung nach Anspruch 1, wobei die Lungenverletzung als Reaktion auf ein biologisches Agens auftritt, und wobei das biologische Agens Pseudomonas pneumonia ist.

6. Verwendung nach Anspruch 1, wobei die Lungenverletzung als Reaktion auf Rauch und Asbest oder beides auftritt.

7. Verwendung nach Anspruch 1, wobei die Kombination aus Antithrombin III und Heparin mit einem Ultraschallzerstäuber verabreicht wird.

8. Verwendung nach Anspruch 1, wobei das Antithrombin III aus Plasma gewonnenes Antithrombin III ist.

9. Verwendung nach Anspruch 1, wobei das Antithrombin III rekombinant produziertes Antithrombin III ist.

10. Verwendung nach Anspruch 9, wobei das rekombinante Antithrombin III transgen produziertes Antithrombin III ist.

11. Verwendung nach Anspruch 1, wobei dem Probanden mehr als eine Dosis des Antithrombins III verabreicht wird.

12. Verwendung nach Anspruch 1, wobei das Antithrombin III in einer Dosis von etwa 10-300 Einheiten/kg Körpergewicht verabreicht wird.

13. Verwendung nach Anspruch 1, wobei das Antithrombin III in einer Dosis von etwa 25-125 Einheiten/kg Körpergewicht verabreicht wird.

14. Verwendung nach Anspruch 1, wobei die Lungenverletzung sowohl mit Antithrombin III als auch mit Heparin behandelt wird.

15. Verwendung nach Anspruch 1, wobei die behandelte Lungenverletzung durch Einatmen von Rauch verursacht wurde.

16. Verwendung nach Anspruch 1, wobei die behandelte Lungenverletzung durch Brandschädigung der Lungen verursacht wunde.

17. Verwendung eines Proteins, das durch ein transgenes DNA-Konstrukt codiert wird, das Antithrombin III aufweist, bei der Herstellung eines Medikaments für die Behandlung von akuter Lungenverletzung.

18. Verwendung eines Proteins, das durch ein transgenes DNA-Konstrukt codiert wird, das Antithrombin III aufweist, bei der Herstellung eines Medikaments für die Behandlung von akuter Lungenverletzung, wobei eine im wesentlichen pharmazeutisch reine Zusammensetzung des Proteins zusammen mit einer wirksamen Menge Heparin verabreicht wird.

19. Verwendung nach Anspruch 1, wobei das Heparin aus Plasma gewonnenes Heparin ist.

20. Verwendung nach Anspruch 1, wobei das Heparin rekombinant produziertes Heparin ist.

21. Verwendung nach Anspruch 20, wobei das rekombinante Heparin transgen produziertes Heparin ist.

22. Verwendung nach Anspruch 1, wobei dem Probanden mehr als eine Dosis des Heparins verabreicht wird.

23. Verwendung nach Anspruch 1, wobei das Heparin in einer Dosis von etwa 10-300 Einheiten7kg Körpergewicht verabreicht wird.

24. Verwendung nach Anspruch 1, wobei das Heparin in einer Dosis von etwa 25-125 Einheiten/kg Körpergewicht verabreicht wird.

## Revendications

1. Utilisation d'antithrombine III et d'héparine dans la fabrication d'un médicament destiné au traitement d'une lésion pulmonaire aiguë, dans laquelle le médicament est administrée en une quantité thérapeutiquement efficace par inhalation.

2. Utilisation selon la revendication 1, dans laquelle la lésion pulmonaire est une lésion pulmonaire aiguë septique.

3. Utilisation selon la revendication 1, dans laquelle la lésion pulmonaire est une syndrome de détresse respiratoire aiguë (SDRA).

4. Utilisation selon la revendication 1, dans laquelle la lésion pulmonaire est présente en réponse à une exposition à un agent viral.

5. Utilisation selon la revendication 1, dans laquelle la lésion pulmonaire est présente en réponse à un agent biologique, cet agent biologique étant Pseudomonas pneumonia.

6. Utilisation selon la revendication 1, dans laquelle la lésion pulmonaire est présente en réponse à au moins un facteur sélectionné parmi la fumée et l'amiante.

7. Utilisation selon la revendication 1, dans laquelle l'association d'antithrombine III et d'héparine est administrée à l'aide d'un nébuliseur à ultrasons.

8. Utilisation selon la revendication 1, dans laquelle l'antithrombine III est une antithrombine III obtenue à partir de plasma.

9. Utilisation selon la revendication 1, dans laquelle l'antithrombine III est une antithrombine III produite par recombinaison.

10. Utilisation selon la revendication 9, dans laquelle l'antithrombine III recombinant est une antithrombine III produite par voie transgénique.

11. Utilisation selon la revendication 1, dans laquelle le sujet reçoit une administration de plus d'une dose de l'antithrombine III.

12. Utilisation selon la revendication 1, dans laquelle l'antithrombine III est administrée à une dose d'environ 10-300 U/kg de poids corporel.

13. Utilisation selon la revendication 1, dans laquelle l'antithrombine III est administrée à une dose d'environ 25-125 U/kg de poids corporel.

14. Utilisation selon la revendication 1, dans laquelle la lésion pulmonaire est traitée à la fois avec ATIII et l'héparine.

15. Utilisation selon la revendication 1, dans laquelle la lésion pulmonaire traitée a été causée par l'inhalation de fumée.

16. Utilisation selon la revendication 1, dans laquelle la lésion pulmonaire traitée a été causée par des brûlures aux poumons.

17. Utilisation d'une protéine codée par une construction d'ADN transgénique comprenant AIIII dans la fabrication d'un médicament destiné au traitement d'une lésion pulmonaire aiguë.

18. Utilisation d'une protéine codée par une construction d'ADN transgénique comprenant ATIII dans la fabrication d'un médicament destiné au traitement d'une lésion pulmonaire aiguë, dans laquelle une composition sensiblement pharmaceutiquement pure de ladite protéine est administrée conjointement avec une quantité efficace d'héparine.

19. Utilisation selon la revendication 1, dans laquelle l'héparine est une héparine obtenue à partir de plasma.

20. Utilisation selon la revendication 1, dans laquelle l'héparine est une héparine produite par recombinaison.

21. Utilisation selon la revendication 20, dans laquelle l'héparine recombinante est une héparine produite par voie transgénique.

22. Utilisation selon la revendication 1, dans laquelle le sujet reçoit une administration de plus d'une dose de l'héparine.

23. Utilisation selon la revendication 1, dans laquelle l'héparine est administrée à une dose d'environ 10-300 U/kg de poids corporel.

24. Utilisation selon la revendication 1, dans laquelle l'héparine est administrée à une dose d'environ 25-125 U/kg de poids corporel.
